# EUROPEAN PATENT SPECIFICATION

(11) **EP 1 945 621 B1**
(45) Date of publication and mention of the grant of the patent: **15.04.2009**
(21) Application number: 06803891.8
(22) Date of filing: 20.09.2006
(51) Int. Cl.: C07D 295/14, C07C 309/65, C07C 309/73, C07C 233/49, C07C 233/47

(54) **PROCESS TO PREPARE AMINO ACID DERIVATIVES**
VERFAHREN ZUR HERSTELLUNG VON AMINOSÄUREDERIVATEN
PROCÉDÉ POUR PRÉPARER DES DÉRIVÉS D'ACIDES AMINÉS

(30) Priority: 21.09.2005 US 718995 P
(43) Date of publication of application: 23.07.2008
(73) Proprietor: DR REDDY'S LABORATORIES (EU) LIMITED, Beverley East Yorkshire HU17 0LD (GB)
(72) Inventor: FOX, Martin, Edward, Royston SG8 6AS (GB); MEEK, Graham, A., Ely (GB)
(74) Representative: Raynor, John
(86) International application number: PCT/US2006/036607
(87) International publication number: WO 2007/038116

(56) References cited:
- WO-A-02/076964
- GABRIEL B ET AL: "Design of Benzamidine-Type Inhibitors of Factor Xa" JOURNAL OF MEDICINAL CHEMISTRY, AMERICAN CHEMICAL SOCIETY. WASHINGTON, US, vol. 41, no. 22, 1998, pages 4240-4250, XP002169719 ISSN: 0022-2623 cited in the application
- MCMURRAY J S ET AL: "Convenient preparation of 4-(tetrazol-5-yl)-phenylalanine for use in Fmoc-based solid-phase peptide synthesis" TETRAHEDRON LETTERS, ELSEVIER, AMSTERDAM, NL, vol. 41, no. 34, 19 August 2000 (2000-08-19), pages 6555-6558, XP004215813 ISSN: 0040-4039 cited in the application
- BAYLE-LACOSTE M ET AL: "SYNTHESIS OF 4-PHOSPHONO- AND 4-(PHOSPHONOMETHYL)-DL-PHENYLALANINE, TWO ANALOGUES OF O-PHOSPHOTOYROSINE" TETRAHEDRON, ELSEVIER SCIENCE PUBLISHERS, AMSTERDAM, NL, vol. 46, no. 23, 1990, pages 7793-7802, XP001084282 ISSN: 0040-4020
- MEHROTRA M M ET AL: "alpha-dicarbonyls as non-charged arginine-directed affinity labels. Novel synthetic routes to alpha-dicarbonyl analogs of the PP60<c-src> SH2 domain-targeted phosphopeptide Ac-TYR (OPO3H2)-glu-glu-ile-glu" BIOORGANIC & MEDICINAL CHEMISTRY LETTERS, OXFORD, GB, vol. 6, no. 16, 20 August 1996 (1996-08-20), pages 1941-1946, XP004135632 ISSN: 0960-894X

## Description

### Field of the invention

The invention relates to an improved process for the preparation of a complex amino acid derivative useful as an intermediate in the synthesis of medicinal products.

### Background to the invention

*N*-(Arylsulfonyl)beta-amino acid derivatives are useful as medicinal products particularly for the treatment of bradykinin-dependent pathologies. See e.g. US2004/0116353. The amino acid derivative (compound **1**),*(R)*-2-*(tert-*butoxycarbonylamino)-3-(4-(*cis*-2,6-dimethylpiperidin-1-yl)methylphenyl)propionic acid, is a key intermediate in the synthesis of *N*-(arylsulfonyl)beta-amino acid derivatives. 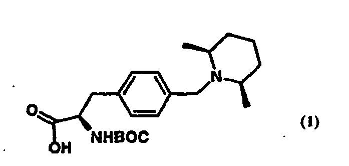 where BOC means tert-butoxycarbonyl. The existing synthetic route to (1), reported in the literature (US2004/0116353A1; see also Tetrahedron Lett., 2000, 41, 6555 and J. Med. Chem., 1998, 41, 4240), is shown in Scheme 1.

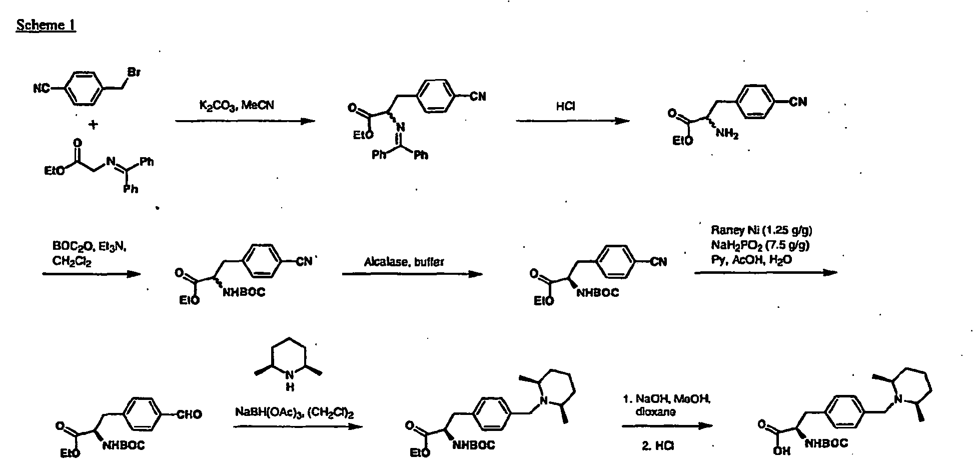

This route suffers from a number of disadvantages, particularly: the requirement for costly starting materials; to obtain enantiomerically enriched material, the use of an enzyme-catalysed resolution process mid-way through the synthesis, resulting in material loss of over 50%; and use of a reductive amination process to introduce the *cis*-2,6-dimethylpiperidine ring, requiring a metal hydride reducing agent. Such processes with hindered amines are prone to side-reactions, particularly when operated at commercial scale. Thus, there is a need for an improved synthetic route to compound **(1)** that provides favourable process economics for large scale commercial operation.

### Summary of the invention

The present invention is an improved route to synthesis of compound 1. The present invention comprises a novel multi-step process to the amino acid derivative **(1)** from (4-hydroxymethyl)benzaldehyde **(2)** including the key steps of: (i) asymmetric hydrogenation, thereby avoiding yield losses associated with the resolution approach and (ii) introduction of the *cis*-2,6-dimethylpiperidine sub-unit by non-reductive *N*-alkylation, thereby avoiding a problematic reductive amination step that is prone to side reactions. In one aspect, the present invention comprises a synthetic route in which step (i) precedes step (ii). In another aspect of the invention, step (ii) precedes step (i). Described another way, the invention is a process for the preparation of the amino acid derivative **(2),** comprising the steps of converting (4-hydroxymethyl)benzaldehyde **(2)** to an enamide intermediate, asymmetric hydrogenation of the enamide intermediate and, either before after the asymmetric hydrogenation step, introduction of the *cis*-2,6-dimethylpiperidine sub-unit by non-reductive *N*-alkylation..

### Description of the invention

In one aspect of the present invention, conversion of the aldehyde **(2)** to the amino acid derivative **(1)** proceeds by the following sequence of steps, as depicted in Scheme 2: 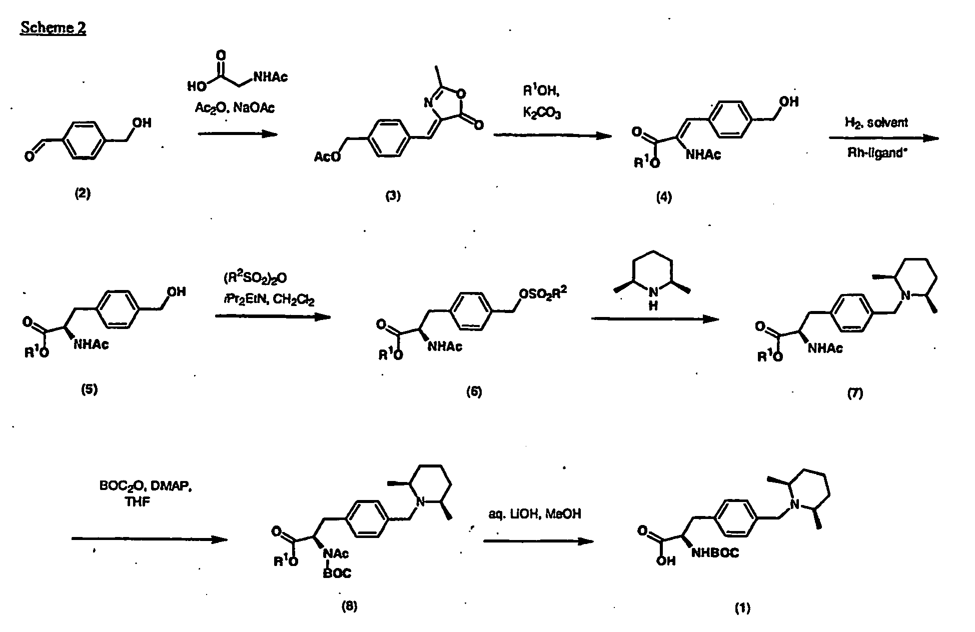
(a) Erlenmeyer condensation to form an azlactone of formula **(3).** Acetic anhydride is the standard reagent in this process but could be replaced with alternative dehydrating agents such as dicyclohexylcarbodiimide, phosphorus pentachloride or thionyl chloride. The process requires a moderately polar organic solvent, commonly ethyl acetate, but a number of alternative solvents are also suitable, for example tetrahydrofuran, 1,2-dimethoxyethane, 1,4-dioxane, acetonitrile and mixtures thereof. The process also requires a mild base, commonly sodium acetate, but a number of alternative bases are also suitable, for example the inorganic bases sodium carbonate, potassium carbonate, sodium phosphate or potassium phosphate and the organic bases triethylamine, ethyldiisopropylamine or pyridine. Heating the reaction mixture under reflux is normally required when using acetic anhydride, but with stronger dehydrating reagents the process may be effected at lower temperatures.
(b) Conversion of azlactone **(3)** to an enamide of formula **(4),** wherein R¹ is methyl or C₂₋₄ linear or branched alkyl. A base is present in order to effect cleavage of the benzylic *O-*acetyl group. Conveniently, potassium carbonate is used, because of its good solubility in methanol. Alternative bases may be used in the process, including sodium carbonate, potassium carbonate, sodium phosphate, potassium phosphate, sodium methoxide and non-nucleophilic organic bases such as 1,8-diazabicyclo[5.4.0]undecene-7 (DBU) and 1,5-diazabicyclo[3.4.0]nonene-5 (DBN).
(c) Asymmetric hydrogenation of enamide **(4)** in the presence of, as catalyst, a rhodium-complex of a chiral phosphorus-containing ligand, to form the amino acid derivative **(5).** Suitable ligands that enable efficient conversion of substrate with high enantioselectivity are identifiable by screening a sufficiently diverse collection of ligands. Choice of solvent is dependent on the particular catalyst but solvents are typically selected from the group including but not limited to lower alcohols (preferably methanol or ethanol), trifluoroethanol, tetrahydrofuran, dichloromethane, toluene or mixtures thereof.
(d) Conversion of **(5)** to the *O*-sulphonyl derivative **(6),** wherein R² is alkyl, haloalkyl or aryl, followed by reaction with *cis*-2,6-dimethylpiperidine, to form the amino acid derivative **(7).** Conveniently, this is accomplished in a one-pot process. For efficient conversion of **(5)** to **(6),** a sulfonic anhydride reagent is required in combination with a tertiary amine base such as ethyldiisopropylamine, triethylamine or trimethylamine. Unexpectedly, alternative reagents that generate nucleophilic counterions are unsuitable. For example, methanesulfonyl chloride generates an alkyl chloride as a reaction byproduct, which is unreactive to displacement by *cis*-2,6-dimethylpiperidine. Any moderately polar aprotic organic solvent is a suitable solvent for the process, for example dichloromethane, tert-butyl methyl ether, toluene, tetrahydrofuran, 1,2-dimethoxyethane, 1,4-dioxane, ethyl acetate or mixtures thereof. Temperature control is important due to the high reactivity of the sulfonic anhydride reagent. Typically this requires a reaction temperature of less than 40°C and conveniently in range of 0-25°C.
**(e)** Conversion of **(7)** to *N*-diprotected derivative **(8),** followed by selective deprotection to remove the *N*-acetyl group and to hydrolyse the ester. Di-*tert*-butyl-dicarbonate, Boc₂O, is the standard reagent for introduction of the *N*-Boc group, although alternative reagents such as Boc-ON=C(CN)Ph, Boc-ONH₂ or Boc-OCH(Cl)CCl₃ can also be used. Selective removal of the *N*-Ac group can be effected with a hydroxide base in the presence of water or an alcohol. The hydroxide base may be replaced by an esterase or other hydrolase enzyme in order to achieve mild hydrolysis of the ester.

Preferred embodiments of this aspect of the present invention can be characterised as follows:
- In step (b), R¹ is methyl or ethyl. More preferably R¹ is methyl.
- In step (c), the chiral phosphorus-containing ligand is a diphosphine, more preferably a bis(2,5-dialkylphospholane) and most preferably is (*R*,*R*)-Ethyl-DuPhos. In contrast to a similar process reported by Chen et al. (Bioorg. Med. Chem. Lett., 2000,10, 729) involving asymmetric hydrogenation of the *O*-TBDMS, N-CBz analogue of **(4),** step (c) does not require protection of the hydroxyl group.
- In step (d), R² is methyl.
- In step (e), for hydrolysis of the ester the hydroxide base is lithium hydroxide and reaction temperature is less than 25°C, more preferably in the range 0-20°C.

In another aspect of the present invention, conversion of the aldehyde **(2)** to the amino acid derivative **(1)** proceeds by the following sequence of steps, as depicted in Scheme 3: 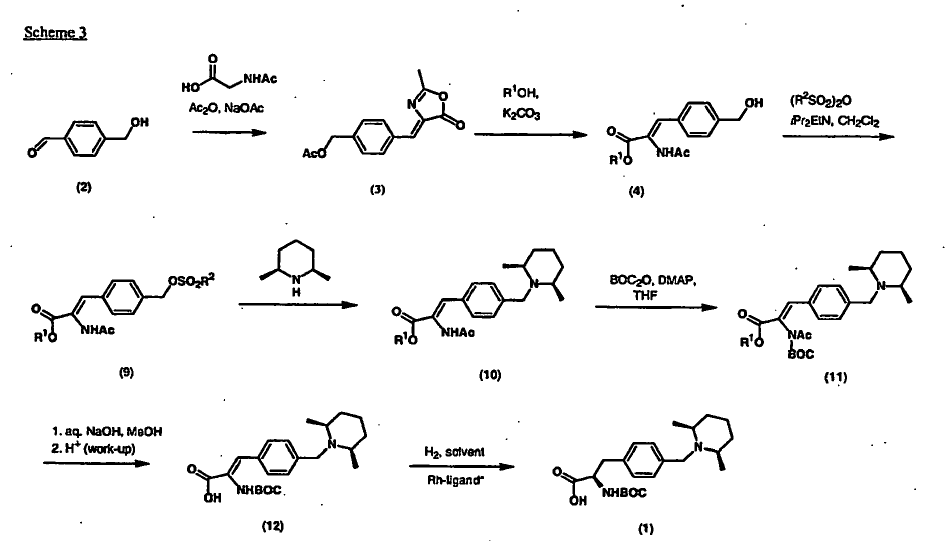
(a) Erlenmeyer condensation to form an azlactone of formula **(3),** as per the sequence described previously.
(b) Conversion of azlactone **(3)** to an enamide of formula **(4),** wherein R¹ is methyl or C₂₋₄ linear or branched alkyl, as per the sequence described previously.
(c) Conversion of enamide **(4)** to the *O*-sulfonyl derivative **(9),** wherein R² is alkyl, haloalkyl or aryl, followed by reaction with *cis*-2,6-dimethylpiperidine, to form the enamide **(10).** The conjugative effect of the *para*-substituent permits use of a broader range of leaving groups than for the conversion of **(5)** to **(7)** in the sequence described previously, such that if desired, the O-sulfonyl leaving group may be replaced by a halide;
(d) Conversion of **(10)** to *N*-diprotected derivative **(11),** followed by selective deprotection to remove the *N*-acetyl group and to hydrolyse the ester. Selective removal of the *N*-Ac group can be effected with a hydroxide base in the presence of an alcohol or other organic solvent, optionally with water as cosolvent. For convenience, the hydroxide base is either sodium hydroxide or potassium hydroxide.
(e) Asymmetric hydrogenation of *N*-Boc enamide **(12)** in the presence of, as catalyst, a rhodium- complex of a chiral phosphorus-containing ligand, to form the amino acid derivative **(1).**

Preferred embodiments of this aspect of the present invention can be characterised as follows:
- In step (b), R¹ is methyl or ethyl. More preferably R¹ is methyl.
- In step (c), R² is methyl and, as depicted, the OH group in **(4)** is derivatized by treatment with methanesulfonic anhydride.
- In step (e), the chiral phosphorus-containing ligand is a diphosphine, more preferably a bis(phospholane) and most preferably is (*R,R*)-Methyl-BPE.

Both routes to the amino acid derivative (1) embodied by the present invention offer advantages over the prior art. In using a catalytic asymmetric step (instead of resolution of a racemic intermediate) and a higher-yielding reaction for introduction of the piperidine group (*N*-alkylation instead of reductive amination) this synthetic approach is more efficient than the existing route. The starting material for both routes, (4-hydroxymethyl)benzaldehyde **(2),** readily prepared from terephthalaldehyde in one step, is a much cheaper material than compounds used in the existing synthesis of **(1)** and in the synthesis of the *O*-TBDMS, *N-*CBz analogue of enamide **(4).**

Both routes to the amino acid derivative **(1)** proceed by novel intermediates. In the first route described (Scheme 2), the enamide **(4)** and enantiomerically enriched intermediates **(5), (6), (7)** & **(8)** are novel compounds. In the second route described (Scheme 3), the enamides **(9), (10), (11)** & **(12)** are novel compounds.

Variants of the present invention will be recognizable to those skilled in the art, specifically the ability to apply the same synthetic approach to other enantiomerically enriched arylalanine derivatives substituted with CH₂-(heterocycle) groups, especially groups incorporating nitrogen-containing heterocycles.

The invention is further illustrated by the following examples.

### Example 1

### Preparation of (4-hydroxymethyl)benzaldehyde (2)

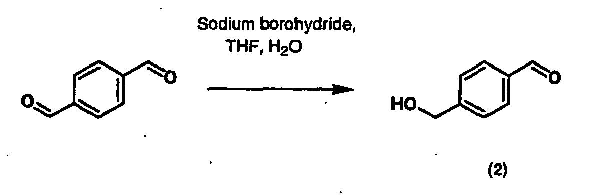

To a 2L three neck flask, fitted with an overhead agitator, thermometer and condenser, terephthalaldehyde (85.8 g) and THF (500 ml) were charged to give a pale yellow solution. Sodium borohydride (8.75 g) was dissolved in water (35 ml) and the resultant solution was charged to the three neck flask over 20 minutes maintaining the internal temperature below 30°C. The reaction mixture was stirred for a further 2 hours; reaction was complete by GC (dialdehyde 0.5%, monoaldehyde 58.9%, diol 36.0%). Water (500 ml) and toluene (500 ml) were charged with stirring and then the layers were separated. The aqueous layer was further extracted with toluene and then the combined organics were backwashed with water. The organic layer was distilled at ambient pressure until the internal temperature reached 105 °C; approximately 600 ml of distillates were collected. The mixture was cooled to below 30°C and then hexane (500 ml) was charged before cooling to 0-5 °C in order to precipitate the product from solution. The solid product was isolated by vacuum filtration and the solid cake was washed with a 50/50 mixture of toluene and hexane (200 ml total) and then dried at 40-45 °C under vacuum to give 69.02 g of **(2).** The solid melted in the vacuum oven. The product purity by GC was 66.2% with 27.9% of the diol impurity present. ¹H NMR (400 MHz, CDCl₃) δ ppm 10.00 (1H, s), 7.88 (2H, d, *J* = 8.0 Hz), 7.53 (2H, d, *J*= 8.0 Hz), 4.80 (2H, s) and 2.1 (1H, br s); ¹³C NMR (100 MHz, CDCl₃) δ ppm 192.7, 148.4, 135.9, 130.4, 127.0 and 64.8.

### Example 2

### Preparation of 4-[(Z)-(2-methyl-5-oxo-1,3-oxazol-4(5H)-ylidene)methyl]benzyl acetate (3)

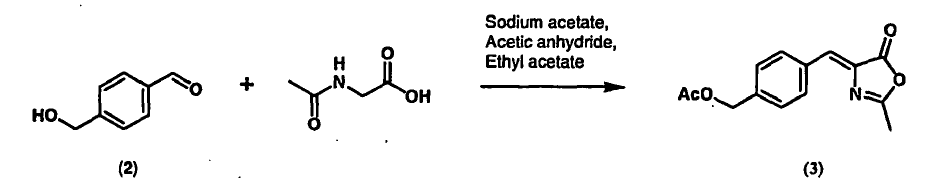

To a 2 L three neck flask, fitted with an overhead agitator, thermometer and condenser sodium acetate (59.2 g), (4-hydroxymethyl)benzaldehyde **(2)** (69.0 g), ethyl acetate (100 ml) and acetic anhydride (120ml) were charged. The slurry was heated to reflux (104-106 °C) and maintained at reflux for 15 minutes before cooling to below 40 °C. *N*-acetylglycine (42.2 g) and ethyl acetate (100 ml) were charged, the resultant mixture was heated to reflux (93-95 °C) and maintained for 18 hours. The batch was cooled to below 60 °C at which point it solidified and water (400ml) was added, the resultant solid was isolated by vacuum filtration, the slurry washed in water (300 ml), then washed in MTBE (300 ml) and then displacement washed with MTBE (100 ml). The yellow solids were dried under vacuum at 45 °C to give 56.2 g of (3); purity by GC was 96.1%; ¹H NMR (400 MHz, CDCl₃) δ ppm 8.08 (2H, d, *J*= 7.8 Hz), 7.42 (2H, d, *J*= 7.8 Hz), 7.13 (1H, s), 5.14 (2H, s), 2.41 (3H, s) and 2.13 (3H, s); ¹³C NMR (100 MHz, CDCl₃) δ ppm 171.2, 168.1, 166.7, 139.4, 133.4, 133.2, 132.7, 131.1, 128.7, 66.1, 21.4 and 16.1. 1.

### Example 3

### Preparation of methyl (Z)-2-(acetylaminol-3-[4-(hydroxymethyl)phenyl]progenoate (4)

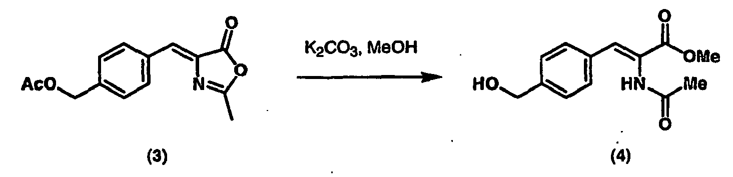

Potassium carbonate (89 mg, 0.64 mmol) was suspended in methanol (12 ml). The azlactone **(3)** (3.40 g, 13.1 mmol) was added in portions over 45 minutes, then the brown solution was stirred for another 1 h. The solution was neutralized from pH 12.3 to pH 6-7 with Dowex 50WX-8-200 ion exchange resin, then the resin was removed by filtration. The solvent was evaporated and the compound was purified by recrystallisation from ethyl acetate (about 20 ml) to give the enamide **(4)** as a yellow, granular solid (3.03 g, 91.8%); ¹H NMR (400 MHz, acetone-d₆) δ ppm 8.7 (1H, brs), 7.60 (2H, d, *J* = 8.0 Hz), 7.39 (2H, d, *J* = 8.0 Hz), 7.24 (1H, s), 4.65 (2H, s), 4.4 (1H, brs), 3.75 (3H, s) and 2.09 (3H, s); ¹³C NMR (100 MHz, acetone-d6) δ ppm 170.2, 170.1, 166.9, 145.1, 133.7, 132.6, 131.0, 127.8, 127.6, 127.4, 64;6, 52.8 and 23.2.

### Example 4

### Preparation of methyl (2R)-2-(acetylamino)-3-[4-(hydroxymethyl)phenyl]propanoate (5)

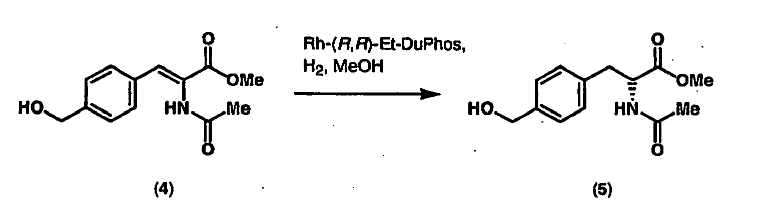

To a 50ml Parr hydrogenation vessel **(4)** (1.25g) and methanol (20 ml) were charged and the vessel was sealed and purged. The catalyst [(*R,R*)-Ethyl-DuPhosRh(COD)]BF₄ (0.05 g) was charged and after resealing and purging the vessel the mixture hydrogenated at 100PSI until uptake of hydrogen gas ceased. The clear yellow solution was concentrated under vacuum at 40°C to give pale yellow solids in methanol, toluene (50 ml) was charged and the remainder of the methanol was removed at 40°C. The solid was isolated by vacuum filtration washed with toluene (20 ml) and dried to 45°C to give 1.1 g of (5). The enantiomeric excess of the product was measured and found to be greater than 98%. ¹H NMR (400 MHz, CDCl₃) δ ppm 7.30 (2H, d, *J*= 8.0 Hz), 7.08 (2H, d, *J*= 8.0 Hz), 5.88 (1H, d, *J* = 6.8 Hz), 4.92-4. 86 (1H, m), 4.68 (2H, s), 3.75 (3H, s), 3.17 (1H, dd, *J*= 13.6, 5.6 Hz), 3.10 (1H, dd, *J*= 13.6, 5.4 Hz) and 2.00 (3H, s); ¹H NMR (400 MHz, acetone-d₆) δ ppm 7.40 (1H, d, *J* = 6.4 Hz), 7.28 (2H, d, *J* = 7.6 Hz), 7.19 (2H, d, *J* = 7.6 Hz), 4.71-4.65 (1H, m), 4.60 (2H, s), 3.10 (1H, dd, *J*=13.2, 5.2 Hz), 2.95 (1H, *J*= 13.2, 8.2 Hz), 2.9 (1H, brs) and 1.88 (3H, s); ¹³C NMR (100 MHz, acetone-d₆) δ ppm 173.3, 170.5, 142.2, 136.9, 130.2, 127.8, 64.7, 54.9, 52.6, 38.4 and 23.0.

### Example 5

### Preparation of methyl (2R)-2-acetylamino-3-(4-{[(2R,6S)-2,6-dimethylpiperidin-1-yl]methyl}phenyl)propanoate (7)

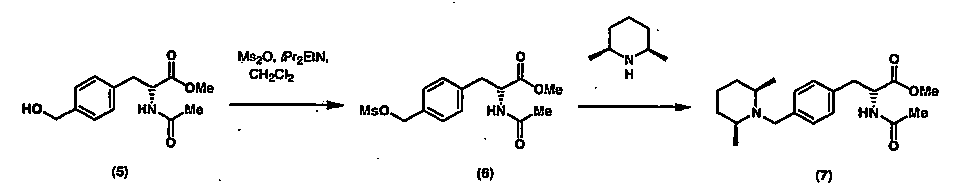

To a 250 ml three neck flask, fitted with an overhead agitator, thermometer, and addition funnel, **(6)** (20.0 g), diisopropylethylamine (16.3 g) and dichloromethane (80 ml) were charged. Methanesulfonic anhydride (18.6 g) was dissolved in dichloromethane (70 ml) and charged to the addition funnel, then charged to the batch over 20 minutes maintaining the temperature below 25° C. The resultant orange solution, containing *O*-mesylate **(6)** was stirred for 3 hours then *cis*-2,6-dimethylpiperidine (18.6g) was charged, the mixture was stirred at ambient temperature for 5 hours then further methanesulfonic anhydride (4.5 g) and *cis*-2,6-dimethylpiperidine (9.0 g) were charged. The dark orange solution was stirred overnight at ambient temperature. The product was extracted into 20% w/w aqueous citric acid (120 g), washed with dichloromethane (70 ml) then basified with 50% aqueous potassium carbonate solution (100 g). The product oil was extracted into dichloromethane (2 x 100 ml) then distilled at 40°C to give 18.5 g of (7), a pale orange viscous oil. Purity by HPLC was 93%. ¹H NMR (400 MHz, CDCl₃) δ ppm 7.30 (2H, d, *J*= 7.8 Hz), 7.00 (2H, d, *J*. = 7.8 Hz), 5.97 (1H, d, *J*= 8.0 Hz), 4.89-4.84 (1H, m), 3.77 (2H, s), 3.71 (3H, s), 3.14-3.04 (2H, m), 2.46-2.44 (2H, m), 1.67-1.62 (1H, m), 1.57-1.55 (2H, m), 1.32-1.28 (3H, m) and 1.05 (6H, d, *J*= 5.6 Hz); ¹³C NMR (100 MHz, CDCl₃) δ ppm 172.6, 170.0, 141.3, 133.8, 129.2, 128.7, 57.7, 53.7, 53.5, 52.7, 37.9, 35.0, 24.7, 23.6 and 22.6.

### Example 6

### Preparation of 2(R)-(tert-butoxycarbonyl-amino)-3-(4-(cis-2,6-dimethylpiperidin-1-yl)-methyl-phenyl)-propionic acid (1)

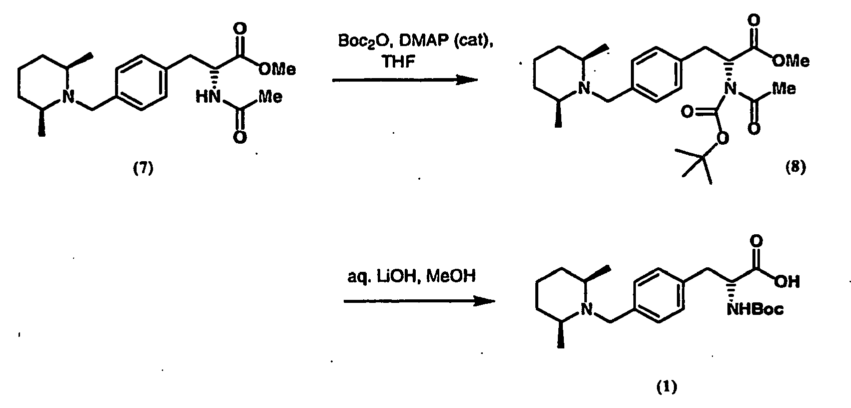

The *N*-acetyl substrate **(7)** (605 mg, 1.75 mmol) and di-*tert*-butyl dicarbonate (573 mg; 2.63 mmol) were dissolved in THF (3 ml). 4-dimethylaminopyridine (21 mg, 0.175 mmol) was added. The solution was stirred for 22 h. More di-*tert*-butyl-dicarbonate (115 mg, 0.53 mmol) was added and stirring was continued for 16 h. The solvent was evaporated and the residue, comprising the intermediate *N*-Boc *N-*acetyl methyl ester **(8),** was dissolved in MeOH (8 ml). At this point the solution was divided in two and half was converted into compound (1) using the following procedure: The methanol solution was cooled in an ice bath and 1M aqueous lithium hydroxide solution (0.9 ml) was added. After 3 h more 1M LiOH (0.9 ml) was added and the mixture was stirred for 1h. MeOH was evaporated under reduced pressure. The aqueous residue was diluted with water (3 ml) and extracted with DCM (2 × 5 ml). The aqueous phase was acidified with 1 M citric acid (to pH 5.8) and then extracted with MTBE (5 ml). The aqueous phase was evaporated under reduced pressure and extracted with hot ethyl acetate (2 × 10 ml). The solvent was evaporated to give 242 mg (71%) of the (R)-N-Boc amino acid (1) as a pale yellow foam, ee >98%. ¹H NMR (400 MHz, CDCl₃) δ ppm 7.26 (2H, d, *J* = 7.8 Hz), 7.04 (2H, d, *J*= 7.8 Hz), 5.47 (dd, *J*= 10.2, 6.0 Hz), 3.76 (2H, s), 3.73 (3H, s), 3.40 (1H, dd, *J* = 14.0, 6.0 Hz), 3.14 (1H, dd, *J* = 14.0, 10.2 Hz), 2.45-2.42 (2H, m), 2.28 (3H, s), 1.64-1.62 (1H, m), 1.57-1.54 (2H, m), 1.43 (9H, s), 1.31-1.26 (3H, m) and 1.05 (6H, d, *J* = 5.6 Hz); ¹³C NMR (100 MHz, CDCl₃) δ ppm 173.0, 171.3, 152.4, 140.6, 135.4, 129.4, 128.4, 84.2, 57.6, 57.5, 57.4, 53.7, 52.7, 35.7, 35.1, 35.1, 28.3, 26.7, 24.7 and 22.6.

### Example 7

### Preparation of (Z)-2-N-BOC-amino-3-(4-{[(2R,6S)-2,6-dimethylpiperidin-1-yl]-methyl}phenyl)propenoic acid (12)

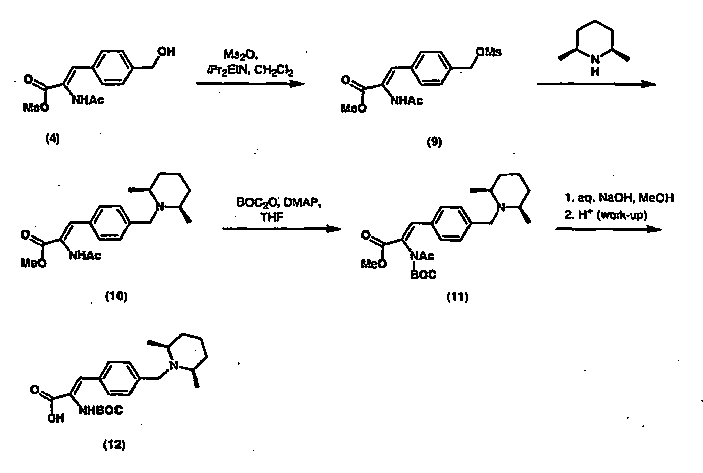

To a 250 ml three neck flask, fitted with an overhead agitator and thermometer, enamide **(4)** (5.0 g), DCM (40 ml) and diisopropylethylamine (4.2 g) were charged. A solution of methanesulfonic anhydride (4.5 g) in DCM (20ml) was added to the batch maintaining the temperature below 25 °C. The mixture was stirred for 3 hours at ambient temperature; conversion to the mesylate **(8)** was 80% by HPLC. *cis*-2,6-Dimethylpiperidine (6.8 g) was charged and the solution stirred for 21 hours. Further methanesulfonic anhydride (0.5 g) and *cis*-2,6-dimethylpiperidine (0.7 g) were required to give 84% conversion by HPLC after a further 3 hours. The organic solution was washed with sodium carbonate (4 g) in water (40 ml) then concentrated under vacuum to remove the bulk of the solvent. Hexane (60 ml) was charged and the mixture screened, then stirred at ambient temperature to precipitate. The product was isolated by vacuum filtration and washed with hexane (20 ml) then dried under vacuum at 45°C to give 4.27 g of **(10).** Purity by HPLC was 96.5%.

To a 100ml two necked flask, fitted with a thermometer and magnetic stirrer, **(10)** (3.0 g), DMAP (30 mg) and THF (15 ml) were charged. A solution of di-*tert*-butyl dicarbonate (2.8 g) in THF (5 ml) was made and then charged to the batch at 20-25 °C. The solution was further stirred for 3 hours, conversion to *N-*Boc *N*-acetyl ester **(11)** was complete by HPLC. Sodium hydroxide (1.4 g) in methanol (15 ml) was charged and the mixture was stirred overnight; conversion was 70%. The methanol and THF solvent were removed by vacuum distillation and during the distillation the reaction also progressed to completion. The pH was adjusted to 5.8 with 36% hydrochloric acid and the resultant liquid was distilled to an oil. IPA (40 ml) was charged and the solvent was removed under vacuum to a concentrated slurry, IPA (40 ml) was charged and the solvent was removed under vacuum to a concentrated slurry. IPA (25 ml) was charged and the mixture was screened to remove inorganic solids, the filtrates were then stirred over the weekend to precipitate. The product was isolated by vacuum filtration and washed with IPA (10 ml) and then dried under vacuum at 45°C to give 1.9 g of (12). Purity by HPLC was greater than 98%. ¹H NMR (400 MHz, DMSO-d₆) δ ppm 7.62 (1H, br s), 7.21 (4H, s), 6.81 (1H, s), 3.67 (2H, s), 2.45- . 2.38 (2H, m), 1.61-1.52 (3H, m), 1.32-1.16 (12 H, m) and 0.97 (6H, d, *J*= 2.4 Hz).

### Example 8

### (2R)-2-(tert-butoxycarbonyl-amino)-3-(4-{[(2R,6S)-2,6-dimethylpiperidin-1-yl] methyl}phenyl)propanoic acid (R)-(1); alternative preparation from (12)

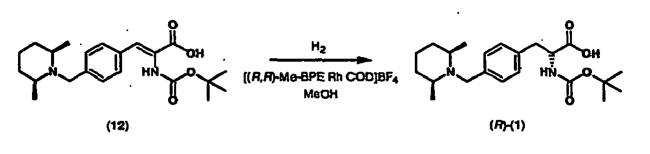

The enamide **(15)** (100 mg, 0.26 mmol) and [(*R*,*R*)-Me-BPE Rh COD]BF₄ (1.4 mg, 0.0026 mmol) were charged to a glass-liner and secured in a Baskerville 10-well autoclave. The vessel was charged with nitrogen to 10 bar and then vented. This charge/vent cycle was repeated twice. Deoxygenated methanol (4 ml) (purged with nitrogen for one hour) was then added and the vessel was charged with hydrogen to a pressure of 10 bar and then vented. This charge/vent cycle was repeated two times. The vessel was then charged with hydrogen to 10 bar and heated via an autocontroller to 40°C. After 17 hours, the vessel was cooled to room temperature and vented. Analysis of the reaction mixture indicated >95% conversion (¹H NMR) and 92% ee. The spectral properties (¹H and ¹³C NMR, HPLC) were identical with **(1)** prepared as described in Example 6.

## Claims

1. A process for the preparation of the amino acid derivative **(1)** from (4-hydroxymethyl)benzaldehyde **(2),** which includes the key steps of asymmetric hydrogenation of an enamide intermediate and introduction of the piperidine sub-unit by non-reductive *N*-alkylation.

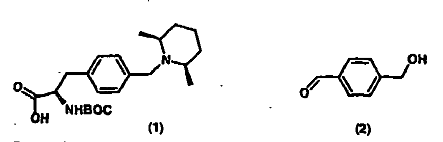

2. A process according to claim 1, which comprises the following steps:
(a) Erlenmeyer condensation of aldehyde **(2)** with *N*-acetylglycine to form an azlactone of formula **(3);**
(b) Conversion of azlactone **(3)** to an enamide of formula **(4),** wherein R¹ is methyl or C₂-C₄ linear or branched alkyl, by reaction with an alcohol R¹OH in the presence of base;
(c) Asymmetric hydrogenation of enamide **(4)** in the presence of, as catalyst, a rhodium- complex of a chiral phosphorus-containing ligand, to form the amino acid derivative **(5);**
(d) Conversion of **(5)** to the *O*-sulphonyl derivative **(6),** wherein R² is alkyl, haloalkyl or aryl, by reaction with a sulfonic anhydride reagent in the presence of a tertiary amine, followed by reaction with *cis*-2,6-dimethylpiperidine, to form the amino acid derivative **(7);**
(e) Conversion of **(7)** to *N*-diprotected derivative **(8),** followed by treatment with a metal hydroxide base in the presence of alcohol, optionally with water as cosolvent, to effect selective removal the *N*-acetyl group and to hydrolyse the ester.

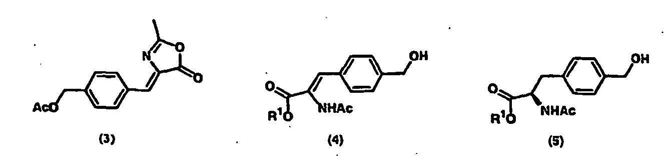

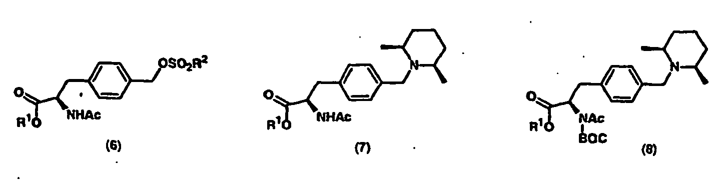

3. A process according to claim 2, wherein in step (b), R¹ is methyl or ethyl.

4. A process according to claim 3, wherein R¹ is methyl.

5. A process according to claim 2, wherein in step (c), the chiral phosphorus-containing ligand is a bis(2,5-dialkylphospholane).

6. A process according to claim 5 wherein the bis(2,5-dialkylphospholane) is (*R*,*R*)-Ethyl-DuPhos.

7. A process according to claim (2) wherein in step (d), R² is methyl and the OH group in (5) is derivatized by treatment with methanesulfonic anhydride.

8. A process according to claim (2) wherein in step (e), the hydroxide base is lithium hydroxide.

9. A compound as utilized in the process according to claim 2 and selected from the group consisting of the enamide **(4)** and enantiomerically enriched intermediates **(5), (6), (7)** & **(8).**

10. A process according to claim 1, which comprises the following steps:
(a) Erlenmeyer condensation of aldehyde **(2)** with *N*-acetylglycine to form an azlactone of formula **(3);**
(b) Conversion of azlactone **(3)** to an enamide of formula **(4),** wherein R¹ is methyl or C₂-C₄ linear or branched alkyl, by reaction with an alcohol R¹OH in the presence of base;
(c) Conversion of enamide **(4)** to the O-sulfonyl derivative **(9),** wherein R² is alkyl, haloalkyl or aryl, followed by reaction with *cis*-2,6-dimethylpiperidine, to form the enamide **(10);**
(d) Conversion of **(10)** to *N*-diprotected derivative **(11),** followed by treatment with a metal hydroxide base in the presence of alcohol, optionally with water as cosolvent, to effect selective removal the *N*-acetyl group and to hydrolyse the ester.
(e) Asymmetric hydrogenation of *N*-Boc enamide **(12)** in the presence of, as catalyst, a rhodium complex of a chiral phosphorus-containing ligand.

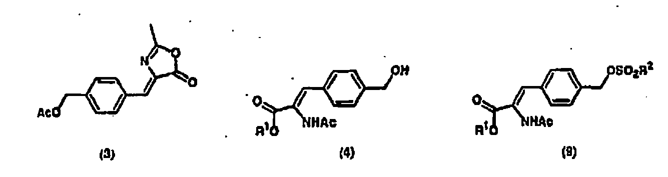

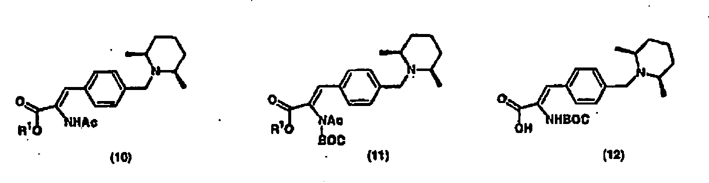

11. A process according to claim 10 wherein in step (b), R¹ is methyl or ethyl

12. A process according to claim 11, wherein R¹ is methyl.

13. A process according to claim 10 wherein in step (c), R² is methyl and the OH group in (4) is derivatized by treatment with methanesulfonic anhydride.

14. A process according to claim 10 wherein in step (e), the chiral phosphorus-containing ligand is a bis(phospholane).

15. A process according to claim 14, wherein the bis(phospholane) is (*R*,*R*)-Methyl-BPE.

16. A compound as utilized in the process according to claim 10 and selected from the group consisting of the enamides **(9), (10), (11)** & **(12).**

## Patentansprüche

1. Verfahren zur Herstellung des Aminosäurederivats (1) aus (4-Hydroxymethyl)benzaldehyd (2), das die wesentlichen Schritte der asymmetrischen Hydrierung eines Enamid-Zwischenprodukts und des Einbringens der Piperidin-Untereinheit durch nichtreduktive N-Alkylierung umfasst.

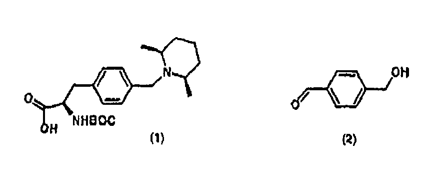

2. Verfahren nach Anspruch 1, das die folgenden Schritte umfasst:
(a) Erlenmeyer-Kondensation von Aldehyd (2) mit N-Acetylglycin, um ein Azlacton der Formel (3) zu bilden;
(b) Umwandlung von Azlacton (3) in ein Enamid der Formel (4), worin R¹ Methyl oder lineares oder verzweigtes C₂-C₄-Alkyl ist, durch Umsetzung mit einem Alkohol R¹OH in Gegenwart einer Base;
(c) asymmetrische Hydrierung von Enamid (4) in Gegenwart, als Katalysator, eines Rhodiumkomplexes eines chiralen phosphorhaltigen Liganden, um das Aminosäurederivat (5) zu bilden;
(d) Umwandlung von (5) in das O-Sulfonylderivat (6), worin R² Alkyl, Halogenalkyl oder Aryl ist, durch Umsetzung mit einem Sulfonsäureanhydrid als Reagens in Gegenwart eines tertiären Amins und anschließende Umsetzung mit *cis*-2,6-Dimethylpiperidin, um das Aminosäurederivat (7) zu bilden;
(e) Umwandlung von (7) in das N-entschützte Derivat (8) und anschließende Behandlung mit einer Metallhydroxidbase in Gegenwart von Alkohol, optional mit Wasser als Hilfslösungsmittel, um das gezielte Entfernen der N-Acetylgruppe zu bewirken und den Ester zu hydrolysieren.

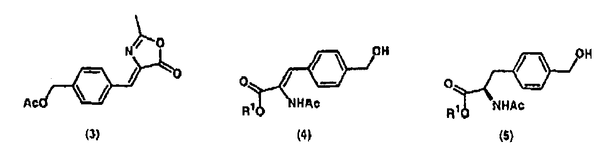

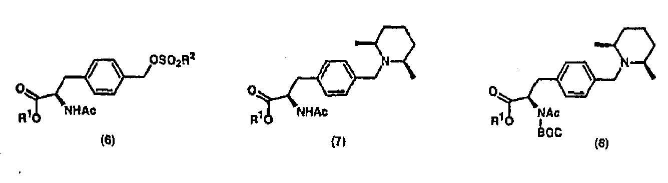

3. Verfahren nach Anspruch 2, wobei in Schritt (b) R¹ Methyl oder Ethyl ist.

4. Verfahren nach Anspruch 3, wobei R¹ Methyl ist.

5. Verfahren nach Anspruch 2, wobei in Schritt (c) der chirale phosphorhaltige Ligand ein Bis(2,5-dialkylphospholan) ist.

6. Verfahren nach Anspruch 5, wobei das Bis(2,5-dialkylphospholan) (R,R)-Ethyl-DuPhos ist.

7. Verfahren nach Anspruch 2, wobei in Schritt (d) R² Methyl ist und die OH-Gruppe in (5) durch Behandlung mit Methansulfonsäureanhydrid derivatisiert ist.

8. Verfahren nach Anspruch 2, wobei in Schritt (e) die Hydroxidbase Lithiumhydroxid ist.

9. Verbindung, die bei dem Verfahren nach Anspruch 2 verwendet wird und ausgewählt ist aus der aus dem Enamid (4) und enantiomer angereicherten Zwischenprodukten (5), (6), (7) und (8) bestehenden Gruppe.

10. Verfahren nach Anspruch 1, das die folgenden Schritte umfasst:
(a) Erlenmeyer-Kondensation von Aldehyd (2) mit N-Acetylglycin, um ein Azlacton der Formel (3) zu bilden;
(b) Umwandlung von Azlacton (3) in ein Enamid der Formel (4), worin R¹ Methyl oder lineares oder verzweigtes C₂-C₄-Alkyl ist, durch Umsetzung mit einem Alkohol R¹OH in Gegenwart einer Base;
(c) Umwandlung von Enamid (4) in das O-Sulfonylderivat (9), worin R² Alkyl, Halogenalkyl oder Aryl ist, und anschließende Umsetzung mit *cis*-2,6-Dimethylpiperidin, um das Enamid (10) zu bilden;
(d) Umwandlung von (10) in ein N-entschütztes Derivat (11) und anschließende Behandlung mit einer Metallhydroxidbase in Gegenwart von Alkohol, optional mit Wasser als Hilfslösungsmittel, um das gezielte Entfernen der N-Acetylgruppe zu bewirken und den Ester zu hydrolysieren;
(e) asymmetrische Hydrierung von N-Boc-Enamid (12) in Gegenwart, als Katalysator, eines Rhodiumkomplexes eines chiralen phosphorhaltigen Liganden.

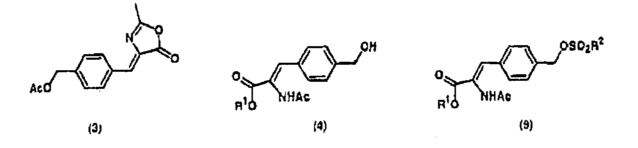

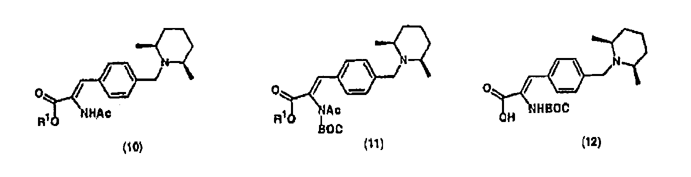

11. Verfahren nach Anspruch 10, wobei in Schritt (b) R¹ Methyl oder Ethyl ist.

12. Verfahren nach Anspruch 11, wobei R¹ Methyl ist.

13. Verfahren nach Anspruch 10, wobei in Schritt (c) R² Methyl ist und die OH-Gruppe in (4) durch Behandlung mit Methansulfonsäureanhydrid derivatisiert ist.

14. Verfahren nach Anspruch 10, wobei in Schritt (e) der chirale phosphorhaltige Ligand ein Bis(phospholan) ist.

15. Verfahren nach Anspruch 14, wobei das Bis(phospholan) (R,R)-Methyl-BPE ist.

16. Verbindung, die bei dem Verfahren nach Anspruch 10 verwendet wird und ausgewählt ist aus der aus den Enamiden (9), (10), (11) und (12) bestehenden Gruppe.

## Revendications

1. Procédé de préparation du dérivé d'acide aminé de formule **(1)** à partir du 4-hydroxyméthyl-benzaldéhyde de formule **(2),** qui comporte les étapes clés d'hydrogénation asymétrique d'un intermédiaire ènamide et d'introduction du fragment de type pipéridine par N-alkylation non-réductrice.

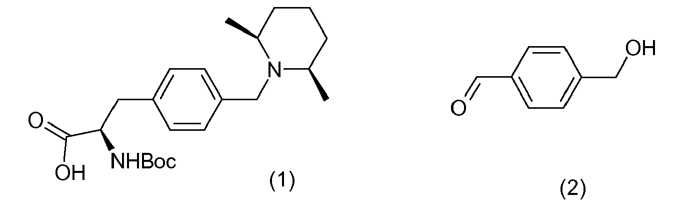

2. Procédé conforme à la revendication 1, qui comporte les étapes suivantes :
a) condensation d'Erlenmeyer de l'aldéhyde de formule **(2)** avec de la N-acétyl-glycine, ce qui donne l'azlactone de formule **(3) ;**
b) conversion de l'azlactone de formule **(3)** en un ènamide de formule **(4),** dans laquelle R¹ représente un groupe méthyle ou alkyle en C₂₋₄ linéaire ou ramifié, par réaction avec un alcool de formule R¹OH en présence d'une base ;
c) hydrogénation asymétrique de l'ènamide de formule **(4)** en présence, en tant que catalyseur, d'un complexe de rhodium et d'un ligand chiral phosphoré, ce qui donne le dérivé d'acide aminé de formule **(5) ;**
d) conversion du dérivé de formule **(5)** en un dérivé O-sulfonylé de formule **(6),** dans laquelle R² représente un groupe alkyle, halogénoalkyle ou aryle, par réaction avec un réactif de type anhydride sulfonique en présence d'une amine tertiaire, puis réaction avec de la cis-2,6-diméthyl-pipéridine, ce qui donne un dérivé d'acide aminé de formule **(7) ;**
e) et conversion du dérivé de formule **(7)** en le dérivé N-diprotégé de formule **(8),** puis traitement effectué avec une base de type hydroxyde de métal en présence d'un alcool et, en option, d'eau qui sert de co-solvant, afin d'éliminer sélectivement le groupe N-acétyle et d'hydrolyser l'ester.

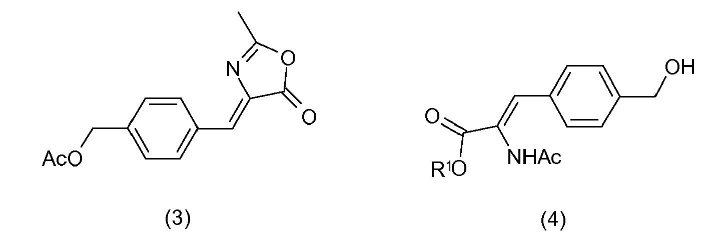

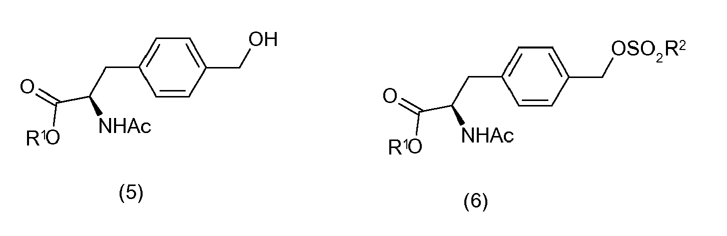

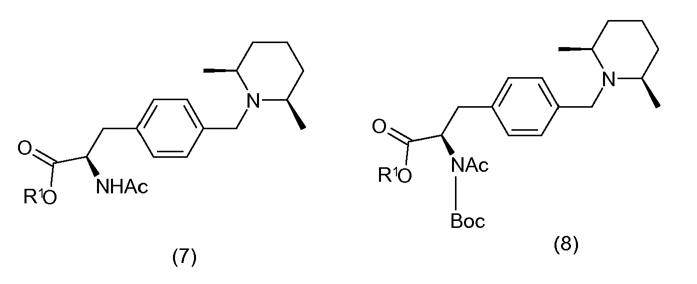

3. Procédé conforme à la revendication 2, dans lequel, dans l'étape (b), R¹ représente un groupe méthyle ou éthyle.

4. Procédé conforme à la revendication 3, dans lequel R¹ représente un groupe méthyle.

5. Procédé conforme à la revendication 2, dans lequel, dans l'étape (c), le ligand chiral phosphoré est un bis(2,5-dialkyl-phospholane).

6. Procédé conforme à la revendication 5, dans lequel le bis(2,5-dialkyl-phospholane) est du (R,R)-éthyl-DuPhos.

7. Procédé conforme à la revendication 2, dans lequel, dans l'étape (d), R² représente un groupe méthyle, et le groupe hydroxyle du dérivé de formule **(5)** est chimiquement modifié par traitement avec de l'anhydride méthane-sulfonique.

8. Procédé conforme à la revendication 2, dans lequel, dans l'étape (e), la base de type hydroxyde est de l'hydroxyde de lithium.

9. Composé employé dans un procédé conforme à la revendication 2 et choisi dans l'ensemble formé par l'ènamide de formule **(4)** et les composés intermédiaires enrichis en énantiomères de formules **(5), (6), (7)** et **(8).**

10. Procédé conforme à la revendication 1, qui comporte les étapes suivantes :
a) condensation d'Erlenmeyer de l'aldéhyde de formule **(2)** avec de la N-acétyl-glycine, ce qui donne l'azlactone de formule **(3) ;**
b) conversion de l'azlactone de formule **(3)** en un ènamide de formule **(4),** dans laquelle R¹ représente un groupe méthyle ou alkyle en C₂₋₄ linéaire ou ramifié, par réaction avec un alcool de formule R¹OH en présence d'une base ;
c) conversion de l'ènamide de formule **(4)** en un dérivé O-sulfonylé de formule **(9),** dans laquelle R² représente un groupe alkyle, halogénoalkyle ou aryle, puis réaction avec de la cis-2,6-diméthyl-pipéridine, ce qui donne un ènamide de formule **(10) ;**
d) conversion du dérivé de formule **(10)** en le dérivé N-diprotégé de formule **(11),** puis traitement effectué avec une base de type hydroxyde de métal en présence d'un alcool et, en option, d'eau qui sert de co-solvant, afin d'éliminer sélectivement le groupe N-acétyle et d'hydrolyser l'ester ;
e) et hydrogénation asymétrique du N-Boc-ènamide de formule **(12)** en présence, en tant que catalyseur, d'un complexe de rhodium et d'un ligand chiral phosphoré.

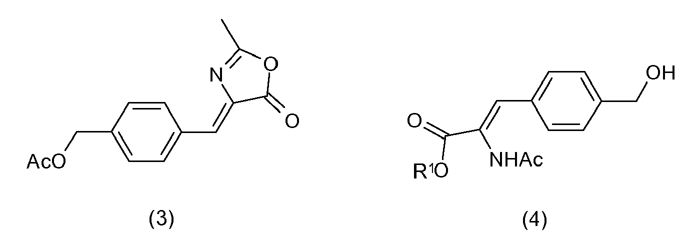

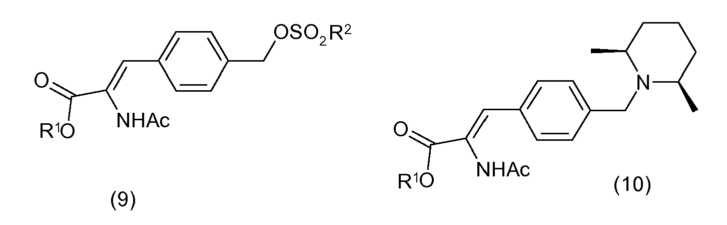

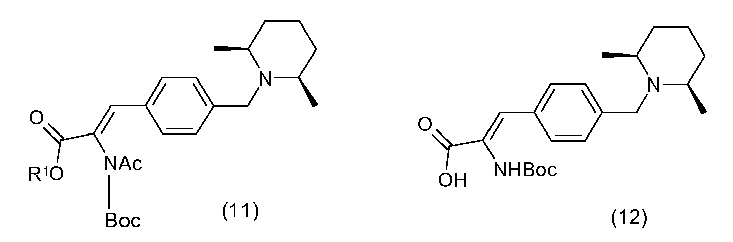

11. Procédé conforme à la revendication 10, dans lequel, dans l'étape (b), R¹ représente un groupe méthyle ou éthyle.

12. Procédé conforme à la revendication 11, dans lequel R¹ représente un groupe méthyle.

13. Procédé conforme à la revendication 10, dans lequel, dans l'étape (c), R² représente un groupe méthyle, et le groupe hydroxyle du dérivé de formule **(4)** est chimiquement modifié par traitement avec de l'anhydride méthane-sulfonique.

14. Procédé conforme à la revendication 10, dans lequel, dans l'étape (e), le ligand chiral phosphoré est un bis(phospholane).

15. Procédé conforme à la revendication 14, dans lequel le bis-(phospholane) est du (R,R)-méthyl-BPE.

16. Composé employé dans un procédé conforme à la revendication 10 et choisi dans l'ensemble formé par les ènamides de formules **(9), (10), (11)** et **(12).**
